(19) Europäisches Patentamt
European Patent Office
Office européen des brevets

(11) Publication number: **0 012 866**
**B1**

(12) **EUROPEAN PATENT SPECIFICATION**

(45) Date of publication of patent specification: **19.01.83**

(21) Application number: **79104750.9**

(22) Date of filing: **29.11.79**

(51) Int. Cl.³: **C 07 D 235/02,**
C 07 D 401/04,
C 07 D 401/10,
C 07 D 403/04,
C 07 D 405/04,
C 07 D 405/10,
C 07 D 409/04,
C 07 D 413/04,
C 07 D 413/10,
A 61 K 31/33

(54) **New 3H-naphtho(1,2-d)imidazoles, processes for preparing them, compounds for use as antiinflammatory and antimicrobial agents and compositions for that use containing them.**

(30) Priority: **21.12.78 GB 4969078**

(43) Date of publication of application:
**09.07.80 Bulletin 80/14**

(45) Publication of the grant of the patent:
**19.01.83 Bulletin 83/3**

(84) Designated Contracting States:
**AT BE CH DE FR GB IT LU NL SE**

(56) References cited:
**DE - B - 1 137 625**
**GB - A - 1 484 615**

**The file contains technical information submitted after the application was filed and not** ***included in this specification***

(73) Proprietor: **GRUPPO LEPETIT S.P.A.**
**8, Via Roberto Lepetit**
**I-20124 Milano (IT)**

(72) Inventor: **Toja, Emilio**
**80, Via Plezzo**
**Milano (IT)**
Inventor: **Omodei-Sale, Amedeo**
**57, Via Papa Giovanni XXIII**
**Voghera (Pa via) (IT)**
Inventor: **Selva, Domenica**
**16, Via Arzaga**
**Milano (IT)**

(74) Representative: **De Carli, Elda et al,**
**PATENT DEPARTMENT c/o Gruppo Lepetit S.p.A.**
**Via Durando 38**
**I-20158 Milano (IT)**

EP 0 012 866 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

New 3-H-naphtho(1,2-d)imidazoles, processes for preparing them, compounds for use as antiinflammatory and antimicrobial agents and compositions for that use containing them

The present invention relates to novel 3H-naphto[1,2-d]imidazole derivatives of the following general formula I

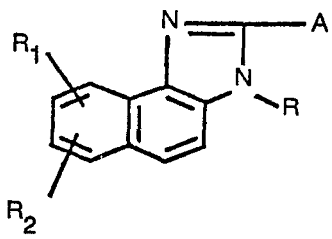

I

wherein R stands for $(C_{1-6})$alkyl or cyclopropyl, $R_1$ and $R_2$, each independently may represent hydrogen, halogen, $(C_{1-4})$alkyl, or $(C_{1-4})$alkoxy and the symbol A represents a heteroaromatic ring selected from furyl, thienyl, pyrrolyl and pyridyl optionally substituted with $(C_{1-4})$alkyl groups, or a phenyl radical optionally substituted with one to three groups independently selected from $(C_{1-4})$alkyl, $(C_{1-4})$alkoxy, amino, mono- and di-$(C_{1-4})$alkylamino, or with one methylenedioxy group; with the proviso that when simultaneously R stands for an ethyl radical, one of $R_1$ and $R_2$ is hydrogen and the other is a methoxy group, A cannot be a 4-dimethylaminophenyl group; and salts therewith of pharmaceutically acceptable acids.

The novel compounds of the present invention possess antiinflammatory, analgesic, antipyretic and antimicrobial utility.

The compounds excluded from the present invention by the above proviso are known from German Patent No. 1.137.625 which reports several thiazole, oxazole and imidazole derivatives with photoconductive properties, that can suitably be employed for electrophotographic reproduction. A compound as in formula I but wherein R is methyl, $R_1$ and $R_2$ are hydrogen and A means a nitro-substituted phenyl group, is known from the article by J. W. Lown and M. H. Akhtor published in Can. J. Chem. *49*, (1971) 1610, where the authors discuss the mechanisms involved in the reaction of 1-nitroso-2-naphthylamine with 3-aroyl-aziridines.

Moroever, other naphthoimidazoles, substituted in the 2-position by an alkyl group, are described in U.S. Patent No. 3,046,116 where it is said that these compounds can be conveniently used in the production of printing plates.

As used herein the term "$(C_{1-4})$alkyl" and the alkyl portion of other hereinlisted radicals containing a $(C_{1-4})$alkyl moiety identifies a straight or branched alkyl radical having from 1 to 4 carbon atoms such as methyl, ethyl, propyl, 1-methylethyl, butyl, 1-methylpropyl, 2-methylpropyl and 1,1-dimethylethyl while the term "$(C_{1-6})$alkyl" designates a straight or branched alkyl radical containing up to 6 carbon atoms such as those listed before, pentyl, 1-ethylpropyl, 1-methylbutyl, 1,1-dimethylpropyl, 1,2-dimethylpropyl, 2,2-dimethylpropyl, hexyl, 1-methylpentyl, 4-methylpentyl, 1,3-dimethylbutyl and 3,3-dimethylbutyl.

The expression "$(C_{1-4})$alkoxy" identifies straight or branched alkoxy radicals having at most 4 carbon atoms which are selected from methoxy, ethoxy, propoxy, 1-methylethoxy, butoxy, 1-methylpropoxy, 2-methylpropoxy, 1,1-dimethylethoxy;

The term and the moiety halogen essentially identifies chloro, bromo and fluoro.

A preferred group of compounds comprises those compounds of formula I wherein R stands for $(C_{1-6})$alkyl or cyclopropyl, $R_1$ and $R_2$ each independently represent hydrogen, halogen $(C_{1-4})$alkyl or $(C_{1-4})$alkoxy and the symbol A represents a phenyl radical optionally substituted with one to three groups independently selected from $(C_{1-4})$alkyl, $(C_{1-4})$alkoxy, amino, mono- and di-$(C_{1-4})$alkylamino, or with one methylenedioxy group; with the proviso that when simultaneously R stands for ethyl, one of $R_1$ and $R_2$ is hydrogen and the other is a methoxy group, A cannot represent a 4-dimethylaminophenyl group; and salts therewith of pharmaceutically acceptable acids.

A second preferred group of compounds comprises those compounds of formula I wherein R stands for $(C_{1-6})$alkyl, $R_1$ and $R_2$ are hydrogen and the symbol A represents a furyl, thienyl, pyridyl or pyrrolyl group, optionally substituted by $(C_{1-4})$alkyl groups; and salts therewith of pharmaceutically acceptable acids. A most preferred group comprises those compounds of formula I wherein R stands for $(C_{1-6})$alkyl, $R_1$ and $R_2$ are both hydrogen and the symbol A represents a phenyl radical optionally substituted with one to three groups independently selected from $(C_{1-4})$alkyl, $(C_{1-4})$alkoxy, amino, mono- and di-$(C_{1-4})$alkylamino; and salts therewith of pharmaceutically acceptable acids.

These acid addition salts are obtained by treating compounds of formula I above with pharmaceutically acceptable acids. As acids suitable for the formation of therapeutically acceptable

salts there may be mentioned, for example, hydrohalide, sulfuric and phosphoric acids, nitric and perchloric acids; aliphatic, alicyclic, aromatic or heterocyclic carboxylic or sulfonic acids, such as formic, acetic, propionic, succinic, glycolic, lactic, malic, tartaric, citric, ascorbic, maleic, hydroxymaleic, pyruvic acid; phenylacetic, benzoic, para-aminobenzoic, anthranilic, para-hydroxybenzoic, salicyclic, para-aminosalicylic or embonic acid, methanesulfonic, ethanesulfonic, hydroxyethanesulfonic, ethylenesulfonic acid; halobenzenesulfonic, toluenesulfonic, naphthalenesulfonic acids or sulfanilic acid.

These or other salts of the new compounds may also be used for purifying the resulting compounds by converting them into salts, isolating the latter and liberating the free compound from them. In view of the close relationship between the new compounds in the free form and in the form of their salts what has been said above and hereinafter with reference to the free compounds concerns also the corresponding salts.

A general method for preparing the novel compounds comprises the condensation between a naphthalenediamine of formula II

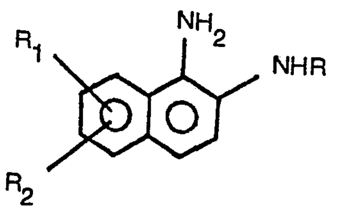

II

wherein R, $R_1$ and $R_2$ are as defined before, and a suitably selected aldehyde of formula ACHO, wherein A is as defined before, to yield an intermediate imidazoline which is subsequently oxidized to end product I. The overall reaction is better illustrated in the following scheme A

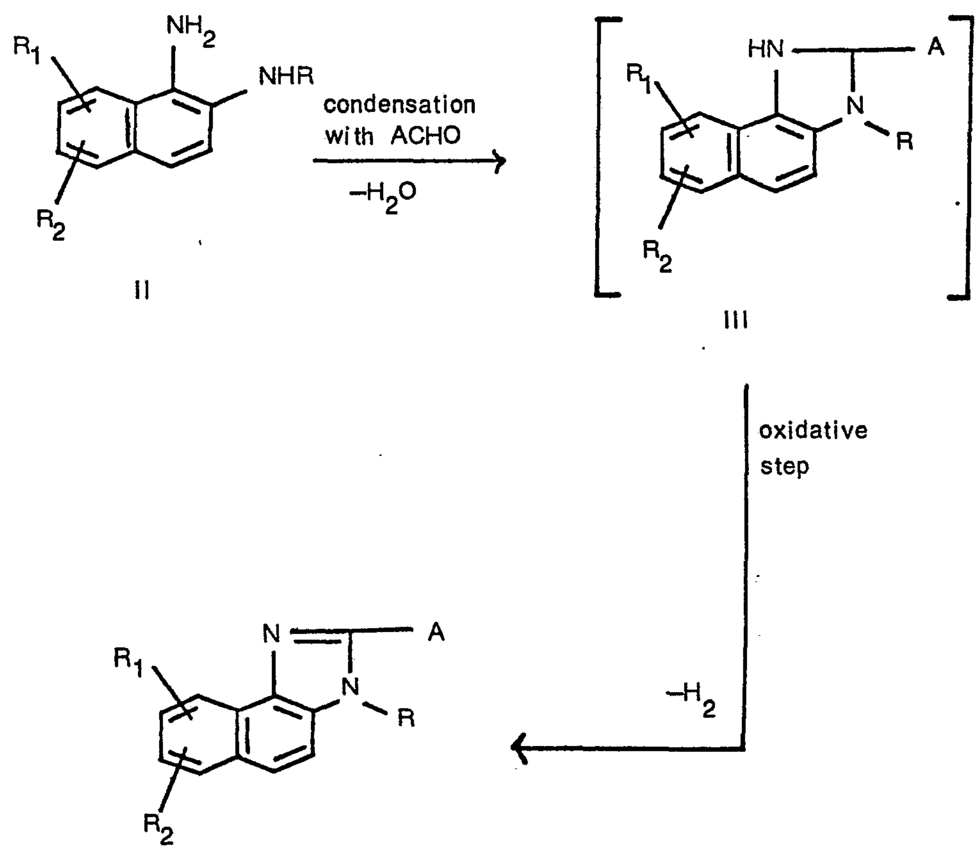


I

wherein the square brackets mean that the intermediate compound placed within them can be further processed without previous separation.

Widely varying conditions can be used to bring about the condensation between the naphthalenediamine and the aldehyde; however rather good results have been obtained adding an equimolecular proportion or a slight excess of the aldehyde to a solution of the compound of formula II in an inert high boiling organic solvent such as for instance xylene, toluene, or cymene and then refluxing the obtained reaction mixture in a Dean-Stark apparatus under inert atmosphere.

As for the oxidative step which in the above scheme is visualized as a simple dehydrogenation, it can be performed in the presence of a mild oxidizing agent, such as for instance manganese dioxide or cupric acetate, or better with a dehydrogenating agent suitably selected from the group of metals or metal oxides generally employed and named as "hydrogenating catalysts" such as for instance Palladium, Platinum, Ruthenium, Rhodium, Platinum dioxide, either in powder form or adsorbed on a charcoal or asbestos carrier, and Raney-Nickel. The obtained reaction products are recovered by conventional procedures which involve filtration of the hot solution and evaporation of the solvent under reduced pressure. Purification of the raw material thus obtained is achieved simply by crystallization or by means of chromatographic techniques.

The starting naphthalenediamine derivatives of formula II are generally novel and may be prepared through different routes; for instance, in J. Org. Chem. *37* (22), 3566 (1972), the synthesis of $N^2$-isopropyl-naphthalen-1,2-diamine is reported through a) nitration of $\beta$-naphthaleneamine to 1-nitro-2-naphthaleneamine, b) exchange of the amino group with a chlorine atom, c) amination with isopropylamine and finally d) reduction of the nitro group to amino.

Other methods moreover can be gathered from the literature considering the particular reactivity of the naphthalene substratum.

The process we have generally employed for preparing the starting naphthalenediamine derivatives involves the reduction of a N-substituted-1-nitroso-2-naphthaleneamine of formula

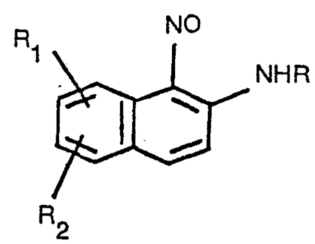

IV

wherein R, $R_1$ and $R_2$ are as defined before, by means of hydrogen gas in the presence of a hydrogenating catalyst. Various hydrogenation catalysts may be employed to bring out the conversion to diamines and generally the same metals and metal oxides employed in the oxidative step of scheme A are preferably used, i.e. Palladium, Platinum, Ruthenium, Rhodium, Platinum dioxide, either in powder form or adsorbed on a carrier, and Raney-nickel. Also the reaction conditions may vary widely since all the catalysts listed above are active, and are preferably used, at room temperature and atmospheric pressure but can suitably be employed also up to 4 atmospheres. Solvents which can conveniently be employed in this reaction are selected from lower aliphatic alcohols such as methanol and ethanol and aromatic hydrocarbons such as for instance benzene, toluene, xylene and cymene.

Alternatively reduction of the N-substituted-1-nitroso-2-naphthaleneamine derivative can also be accomplished by using as reducing agents metals such as tin, zinc or aluminum in an acidic medium according to well known procedures.

The starting nitroso compounds have been synthetized according to the method described by S. T. Morgan and F. P. Evens in J. Chem. Soc. *115*, 1140 (1919), through acid-catalyzed rearrangement of a 2-(N-nitroso-N-substituted)naphthylamine or more conveniently through reaction of primary amines with 1-nitroso-2-naphthol according to E. W. Malmberg and C. S. Hamilton in J. Am. Chem. Soc. *70*, 2415 (1948).

The above reported method for preparing the starting naphtalenediamines II from the corresponding N-substituted-1-nitroso-2-naphthaleneamines is of particular value for any reasons. First of all in fact the reduction reaction does not require drastic conditions but on the contrary it proceeds rapidly at room temperature and atmospheric pressure, secondly the reaction conditions themselves, the solvents and the starting nitroso-compounds employed are particularly safe from the industrial point of view; thirdly the naphthalenediamines thus obtained are not necessarily separated from the reaction mixture and the condensation with the suitably selected aldehyde ACHO can be carried out without any working up of the reaction mixture containing the hydrogenated compound of formula II before adding the aldehyde ACHO. In this case, if separation of the naphthalenediamine is not required, also the reduction of the N-substituted-1-nitroso-2-naphthalenediamines will be carried out in an inert high-boiling organic solvent.

Moreover, since catalyzed reduction, which takes place on the catalyst's surface, is a reversible process, the same catalysts employed for reducing the nitrosonaphthaleneamines can be conveniently employed in the absence of hydrogen, in the dehydrogenation procedure.

The interaction between a naphthalenediamine of formula II and an acid derivative which may be an acyl chloride, anhydride or ester provides an other convenient route to the naphthoimidazoles of the present invention. More particularly, the naphthalenediamine is contacted with a compound of formula ACOX wherein A is as defined before and X may represent

— a chlorine atom,

— a group —$OR_3$ wherein $R_3$ may be the same radical —COA, or a trifluoroacetyl, ethoxycarbonyl or alkyl sulfonyl moiety or,

— a group —$OR_4$ wherein $R_4$ is a methyl or ethyl radical.

This two step reaction involves formation of a mono-acylated naphthalenediamine as the key intermediate according to the following scheme B:

SCHEME B

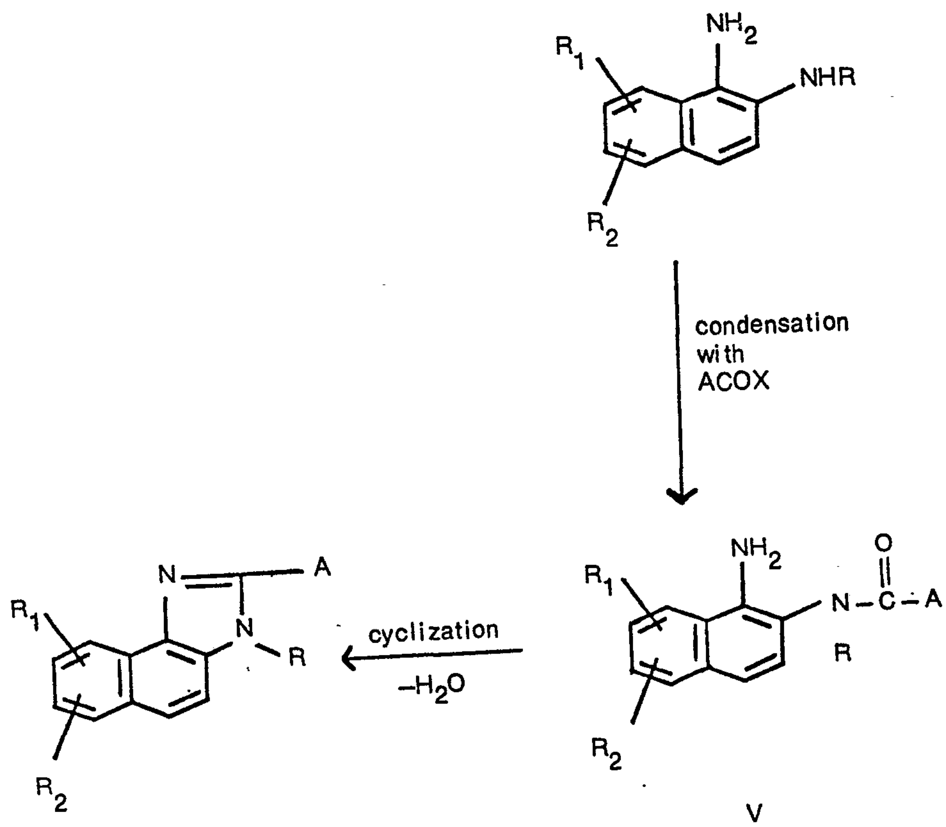


II

V

As for the first step which leads to the intermediate mono-acylated compounds, we found that high yields can be realized when an equimolecular mixture of a naphthalenediamine II and an acid derivative ACOX is dissolved in an anhydrous inert organic solvent selected from lower aliphatic halogenated and aromatic hydrocarbons in the presence of a tertiary organic nitrogen base which should block the inorganic or organic acid which forms during the course of the reaction.

Finally, conversion of the mono-acylated intermediate to the desired end product, through elimination of water, is carried out by refluxing it in an inert organic solvent optionally in the presence of an acidic catalyst such as sulphuric or p-toluenesulphonic acid. Recovery and purification of the end naphthoimidazoles, involves the same conventional procedures already described in the first process. Furthermore some compounds of formula I may be obtained also through chemical modifications of other compounds prepared according to one of the processes outlined in the above reaction schemes. For instance, compounds wherein A is phenyl substituted with $(C_{1-4})$alkoxy are conveniently prepared by reaction of the corresponding hydroxyphenyl derivatives with $(C_{1-4})$alkyl halogenides, tosylates or mesylates.

Again, the compounds wherein A is an aminophenyl group may be easily prepared from the corresponding alkanoylamino and benzoylaminophenyl derivatives by acid hydrolysis. A convenient route leading to monoalkylaminophenyl compounds in high yields, consists in preparing the sodium derivative of the amidic nitrogen atom of a corresponding acylamino derivative, then substituting it by means of an alkylating agent and finally splitting off the protecting acyl group by alkaline hydrolysis.

As stated before, some of the novel compounds of the present invention are active as antiinflammatories, mild analgesics and antipyretics. Moreover, some others possess a fairly good antimicrobial activity particularly against fungi. These biological activities are coupled with a low toxicity since the approximate $LD_{50}$s per os in mice are generally higher than 500 mg/kg. The toxicities were determined according to Lichfield and Wilcoxon, Journ. Pharm. Expt. Ther., *96*, 99, (1949).

The antiinflammatory activity was ascertained by means of several testing methods; in one, the ability of the compounds of the invention to reduce the edema induced in the rat paw by injection of carrageenin was evaluated and the test was performed according to the methodology described by C. A. Winter et al. in Proc. Soc. Exptl. Biol. Med. *111*, 544, (1962).

In another it was investigated the reduction by the test compounds of the weight of the granuloma formed on a cotton pellet implanted subcutaneously in rats, following the method described by Meier et al. in Experientia, *6*, 469, (1950).

In still another some of the compounds were tested in the adjuvant induced arthritis test in rats.

This test which was performed as described by B. B. Newbould in Bri. Journ. Pharmacol., *21*, 127, (1963), is absolutely meaningful, because adjuvant arthritis is one of the best pharmacological tool with which a pharmacologist can investigate compounds as to their possible antiinflammatory activity, owing to the fact that this experimental model of chronic inflammation looks closer like to the conditions of the rheumatoid arthritis of the human pathology (see Pearson, C. M., Arthritis and allied conditions, page 119, Lea and Febiger Publ. 1967 and Pearson C. M., J. Chronic Diseases, *16*, 863 (1963). The compounds were administered to the rats at different dose levels generally corresponding to about one twentieth or one twentyfifth, one tenth and one fifth (the highest dose) of the corresponding toxic doses expressed in $LD_{50}$ values. However, even if the $LD_{50}$ values of the compounds to be tested are higher than 1000 mg/kg, the maximum dose level at which they are administered is generally never higher than 200 mg/kg. These dosages are quite far from the toxic dose. Actually, in the carrageenin-induced oedema test, used as a preliminary screening test, the compounds to be tested were first administered at the highest dose level which, as stated above, corresponds to one fifth of the $LD_{50}$ value or 200 mg/kg if the $LD_{50}$ value is higher than 1000 mg/kg. The compounds displaying an interesting degree of antiinflammatory activity i.e. those causing a percent decrease of the induced edema of about 40 or more, were further tested at lower dosages and then submitted to the granuloma pellet test. The results obtained in these tests are reported in the following Table I:

| Compound of Example No. | $LD_{50}$os | Dose mg/kg os | Percent inhibition of the carrageenin-induced edema | Percent inhibition of the granuloma pellet |
|---|---|---|---|---|
| 1 | 1000 | 50 | 24 | — |
| | | 100 | 37 | — |
| | | 200 | 42 | 24 |
| 3 | 1000 | 50 | 30 | — |
| | | 100 | 44 | — |
| | | 200 | 63 | 33 |
| 4 | >1000 | 50 | 21 | — |
| | | 100 | 37 | — |
| | | 200 | 45 | 40 |
| 5 | ~1000 | 50 | 34 | — |
| | | 100 | 41 | — |
| | | 200 | 53 | 40 |

| Compound of Example No. | $LD_{50}$os | Dose mg/kg os | Percent inhibition of the carrageenin-induced edema | Percent inhibition of the granuloma pellet |
|---|---|---|---|---|
| 11 | >1000 | 50 | 19 | — |
| | | 100 | 31 | — |
| | | 200 | 40 | 31 |
| 12 | 500 | 20 | 32 | — |
| | | 50 | 46 | — |
| | | 100 | 54 | 24 |
| 17 | 500 | 20 | 35 | — |
| | | 50 | 50 | — |
| | | 100 | 64 | 38 |
| 21 | 500 | 20 | 29 | — |
| | | 50 | 37 | — |
| | | 100 | 47 | 33 |
| 25 | >1000 | 50 | 32 | — |
| | | 100 | 45 | — |
| | | 200 | 48 | 45 |
| 30 | >1000 | 20 | 31 | — |
| | | 50 | 45 | — |
| | | 100 | 49 | — |
| | | 200 | 52 | 31 |

The compounds of examples 5, 17 and 25 which gave the best results in the carrageenin-induced edema and in the granuloma pellet tests, were then further tested in the adjuvant arthritis test at a dosage corresponding to 1/5 of their $LD_{50}$ or at 200 mg/kg if the $LD_{50}$ is higher than 1000. The measure of effectiveness of the compounds in this test is given by their ability in reducing the volume of the hind paws of the rats. The results obtained are collected in Table II below:

| Compound of Example No. | Dose mg/kg rats p.o. | % Reduction of the volume of the hind paws over the control |
|---|---|---|
| 5 | 200 | 33 |
| 17 | 100 | 42 |
| 25 | 200 | 53 |

These favorable characteristics are coupled also with interesting analgesic and antipyretic properties which were investigated according to the methods described by Randall et al. in Arch. Int. Pharmacodyn. *111*, 409, (1957) and by Buller et al. in J. Pharm. Pharmacol. *9*, 128, (1957) respectively. It is finally to be noted that the new naphthoimidazoles which are the object of the present invention display a very low ulcerogenic activity which is several times lesser than the one observed with other known and therapeutically used anti-inflammatory substances. The ulcerogenic action was determined according to Thuillier et al. Chim. Ther. *3*, 51, (1968).

Moreover, as anticipated, some of the compounds of the present inventiodn show an appreciable antimicrobial effect mainly against fungi such as various *Trichophyton* species, for instance *Trichophton mentagrophytes, Trychophyton Schoenleinii* and *Trichophyton versicolor.* More particularly, concentrations varying from about 3 to about 25 $\gamma$/ml of the compounds of Examples 5, 9, 11 13, 14, 15 and 16 inhibit the growth of these microorganisms in vitro.

The use of the novel compounds as antiinflammatory and as antimicrobial agents, which is a further specific object of the present invention refers to all industrially applicable aspects and acts of said use including the embodying of the novel compounds or their salts into pharmaceutical compositions.

For antiinflammatory use the compounds of the invention may be administered by different routes. While the preferred routes of administration are oral and rectal, parenteral administration can also be employed. For oral administration the compounds of the present invention are compounded into pharmaceutical dosage forms such as for instance tablets, capsules, elixirs and solutions. Tablets may contain in addition to the therapeutic ingredient the usual additives such inert diluents, for example starch, lactose, kaolin, calcium phosphate and mannitol; binders, for example gelatin, starch sugars, gums, carboxymethylcellulose and polyvinylpyrrolidone; lubricants, for example talc, magnesium stearate, and stearic acid; and the commonly employed disintegrant, coloring, sweetening and flavoring agents. Coated or hard-shell capsules may also be prepared which may contain the same additives indicated above for tablets. Liquid preparations such as elixirs and solutions are prepared by dissolving the active ingredient in an aqueous or non-aqueous pharmaceutically acceptable solvent and may contain also suspending sweetening, flavoring and preservative agents as known in the art.

For rectal administration the compounds are formulated as suppositories wherein the active ingredient is admixed with conventional vehicles such as for example cocoa butter, wax, spermaceti or polyoxyethylenglycols and their derivatives.

The dosage range is from 0.05 to 10.0 g. per day, preferably administered in divided doses. Accordingly, the present invention provides a pharmaceutical composition for antiinflammatory use comprising from 50 to 1000 mg of a compound of the invention as the active ingredient together with a pharmaceutically acceptable carrier.

For antimicrobial use the compounds of the invention are compounded into topical preparations such as ointments, creams and powders in concentration of 0.1 to 10% to be applied one or more times a day as required. Ointments and creams are prepared by incorporating the active ingredient into an ointment base such as for instance a oleaginous base prepared from vegetable and animal fats, a hydrocarbon base prepared from petroleum and wax or, preferably a polyethylenglycol ointment base.

Powders are prepared by mixing the active ingredient in the form of a very finely subdivided powder with a chemically inert vehicle as known in the art.

The following examples illustrate the process of the invention and describe in detail some compounds of general formula I.

Example 1

3-methyl-2-phenyl-3H-naphth[1,2-d]imidazole

11.35 g of benzoyl chloride (0.08 mole) dissolved in 50 cc of methylene chloride is added to a solution of 14.57 g of $N^2$-methylnaphthalene-1,2-diamine (0.084 mole) and 11.3 cc of triethylamine (0.08 mole) in 100 cc of methylene chloride, and the obtained reaction mixture is allowed to stand at room temperature for about one hour. Then it is heated to the reflux temperature for 16 hours, cooled to room temperature and filtered over bleaching earth. The filtrate is diluted with 200 cc of methylene chloride, washed twice with water, once with 5% sodium bicarbonate and then with water up to neutral reaction of the aqueous phase. The methylene chloride solution, dried over sodium sulphate, is concentrated to dryness yielding a residue which taken up with 300 cc of benzene is then poured into a 500 cc flask equipped with a Dean-Stark apparatus. 0.18 g p-toluensulphonic acid is gradually added to this solution heated to the reflux temperature.

After 4 hours, the reaction mixture is cooled to room temperature and filtered. The filtrate washed with water, is dried over sodium sulphate and then concentrated to dryness yielding 13.1 g of the compound of the title. M.p. 127—28°C (from ethanol).

## Example 2

2-(3-methoxyphenyl)-3-methyl-3H-naphth[1,2-d]imidazole

This compound is prepared according to the procedures of example 1 but using 3-methoxybenzoyl chloride (13.75 g) instead of benzoyl chloride. Yield 12.57 g. M.p. 148—149°C (from ethanol).

## Example 3

3-methyl-2-(4-pyridyl)-3H-naphth[1,2-d]imidazole

The compound of the title is prepared by operating according to the procedures of example 1 but using isonicotinoyl chloride hydrochloride (14.25 g) instead of benzoyl chloride. Yield 5.72 g of pure compound. M.p. 158—159°C (from ethanol).

## Example 4

3-methyl-2-(4-ethoxyphenyl)-3H-naphth[1,2-d]imidazole

A solution of 11.16 g (0.06 mole) of 2-methylamino-1-nitroso-naphthalene in 800 cc of toluene is hydrogenated at room temperature and at the atmospheric pressure in the presence of 3 g of Palladium-on-carbon. After one hour, when the theoretical amount of hydrogen has been consumed, 9 cc (0.06 mole) of 4-ethoxy benzaldehyde are added and the obtained reaction mixture is heated to the reflux temperature under an inert atmosphere for about 3 hours. The water which forms during the reaction distillates as binary azeotrope with toluene and is separated through a Dean-Stark apparatus. Then further 1.5 g of 5% Palladium-on-carbon are added and reflux is prolonged for two additional hours. Filtration of the hot solution followed by concentration of the filtrate to dryness under vacuum affords a residue which has been purified by crystallization from ethyl acetate. Yield 14.5 g (80% M.p. 138—9°C.

## Examples 5 to 19

The following compounds are prepared by operating according to the procedures of the foregoing example, by hydrogenating the starting N-methyl-1-nitroso-naphthalenamine, condensing the obtained diamino compound with a suitable selected aldehyde and then dehydrogenating the resulting imidazoline derivative.

5) 2-(4-methoxyphenyl)-3-methyl-3H-naphth[1,2-d]imidazole M.p. 132—134°C (from ethyl acetate)
6) 3-methyl-2-(2-pyridyl)-3H-naphth[1,2-d]imidazole hydrochloride M.p. 231°C (with decomposition) (from ethyl acetate).
7) 3-methyl-2-(6-methyl-2-pyridyl)-3H-naphth[1,2-d]imidazole hydrochloride. M.p. 262°C (decomposition) (from ethanol).
8) 3-methyl-2-(3-pyridyl)-3H-naphth[1,2-d]imidazole hydrochloride. M.p. 245°C (decomposition) (from methanol).
9) 2-(2-furyl)-3-methyl-3H-naphth[1,2-d]imidazole hydrochloride. M.p. 243°C (decomposition) (from isopropanol).
10) 3-methyl-2-(1H-pyrrol-2-yl)-3H-naphth[1,2-d]imidazole. M.p. 283°C (from dioxane)
11 3-methyl-2-(2-thienyl)-3H-naphth[1,2-d]imidazole M.p. 166—8°C (from ethyl acetate)
12) N,N-dimethyl-4-(3-methyl-3H-naphth[1,2-d]imidazol-2-yl)benzenamine. M.p. 136—8°C (from benzene)
13) 3-methyl-2-[4-(isopropoxy)phenyl)-3H-naphth[1,2-d]imidazole. M.p. 144—45°C (from ethyl ether).
14) 3-methyl-2-(4-methylphenyl)-3H-naphth[1,2-d]imidazole. M.p. 135—6°C (from diisopropyl ether)
15) N,N-diethyl-4-(3-methyl-3H-naphth[1,2-d]imidazol-2-yl)benzenamine. M.p. 161—162, 5°C (from ethyl acetate)
16) 2-(1,3-benzodioxol-5-yl)-3-methyl-3H-naphth[1,2-d]imidazole. M.p. 200—201°C (from acetone)
17) 4-(3-methyl-3H-naphth[1,2-d]imidazol-2-yl)-2,N,N-trimethylbenzenamine. M.p. 116—118°C (from methyl-t.butyl ether)
18) 3-methyl-2-(3-methyl-4-methoxyphenyl)-3H-naphth[1,2-d]imidazole. M.p. 104—105°C (from diisopropyl ether).
19) N-[4-(3-methyl-3H-naphth[1,2-d]imidazol-2-yl)phenyl]acetamide. M.p. 271—72°C (from ethanol).

## Example 20

4-(3-methyl-3H-naphth[1,2-d]imidazol-2-yl)benzeneamine

The compound of the title is prepared by acid hydrolysis of the corresponding monoacetyl derivative. More particularly a solution of 5.7 g of the compound of example 19 in 75 cc of 5% HCl is heated to the reflux temperature for about 1½ hours under a nitrogen stream. Then the solution is treated with charcoal and filtered under vacuum. The filtrate brought to basic pH by treating with concentrated ammonium hydroxide, is then cooled and the solid which precipitates is recovered by filtration M.p. 250—2°C.

Example 21

N-methyl-4-(3-methyl-3H-naphth[1,2-d]imidazol-2-yl)benzeneamine

5 g of N-[4-(3-methyl-3H-naphth[1,2-d]imidazol-2-yl)phenyl]acetamide prepared as in Example 19 are dissolved in 80 cc of anhydrous dimethylformamide and to the obtained solution, cooled to a temperature comprised between 0 and 5°C, 0.69 g of 55% NaH are gradually added. After 1½ hours when the evolution of hydrogen gas ceases 1 cc of methyl iodide dissolved in 20 cc of dimethylformamide is dripped in and the reaction mixture is allow to reach room temperature. Then the solution is poured into 1 l. of water and stirred for about 20 minutes; the solid which precipitates is recovered by filtration and dried under vacuum yielding 4.53 g of a mixture of two products one of which does correspond to the desacetylated product. This mixture is dissolved in 300 cc of methyl alcohol and 200 cc of 10% NaOH and refluxed for 6 hours; then methanol is distilled off at atmospheric pressure and the reaction mixture is cooled and diluted with water. The precipitate which forms is recovered by filtration and crystallized from benzene yielding 3.6 g of the compound of the title which melts at 225—27°C.

Example 22

4-(3-methyl-3H-naphth[1,2-d]imidazol-2-yl)phenol

This compound is prepared according to the procedures described under Example 4 bus using 4-hydroxy-benzaldehyde instead of 4-ethoxybenzaldehyde. M.p. > 300°C (from acetic acid).

Example 23

2-(2-methoxyphenyl)-3-methyl-3H-naphth[1,2-d]imidazole

The compound of the title is prepared by following essentially the same procedure described in example 4 but using 2-methoxy-benzaldehyde instead of 4-ethoxybenzaldehyde. Yield 82%. M.p. 132—33°C.

Example 24

4-(3-methyl-3H-naphth[1,2-d]imidazol-2-yl)-N,N,2,6-tetramethylbenzenamine

The compound of the title is prepared by following essentially the same procedures described in example 4 but using 3,5-dimethyl-4-dimethylamino-benzaldehyde instead of 4-ethoxybenzaldehyde.

Purification of the raw product is achieved by trituring the residue with light petroleum, filtering it and crystallizing the obtained product from cyclohexane first and then from ethanol/water. Yield 40%. M.p. 136—7°C.

Example 25

3-(isopropyl)-2-(4-methoxyphenyl)-3H-naphth[1,2-d]imidazole

In a multi-necked 500 cc flask equipped with a mechanical stirrer and a Dean-Stark apparatus a solution of 2 g (0.01 mole) of $N^2$-(isopropyl)naphthalendiamine (known from J. Org. Chem. *37* (22), 3566 (1972)), 1.36 g (0.01 mole) of 4-methoxybenzaldehyde and 300 cc of toluene are heated at the reflux temperature under argon atmosphere for 3 hours. Then 2 g of 5% Palladium on carbon are added and the reaction mixture is heated at the reflux temperature for further three hours. Upon filtering off the catalyst, the filtrate is taken to small volume yielding 2.86 g of a raw product which is recovered by filtration and purified by crystallization from ethyl acetate. From the mother liquors taken to dryness further 1.7 g are obtained which are chromatographed through a silicagel column eluting with benzene: ethyl acetate 95:5. Overall yield 57%. M.p. 162—63°C.

The compound of the title is also prepared by following the same procedure described in example 4 but starting from N-isopropyl-1-nitroso-2-naphthaleneamine instead of N-methyl-1-nitroso-2-naphthaleneamine and using 4-methoxybenzaldehyde instead of 4-ethoxybenzaldehyde. Yield 60%. M.p. 162—63°C (from ethyl acetate).

Example 26

3-butyl-2-(4-methoxyphenyl)-3H-naphth[1,2-d]imidazole

The compound of the title is prepared by following essentially the same procedure described in example 4 but starting from 2-butylamino-2-nitrosonaphthalene instead of 2-methylamino-1-nitrosonaphthalene and employing 4-methoxybenzaldehyde instead of 4-ethoxybenzaldehyde. Yield 53%. M.p. 99,5—100,5°C.

Example 27

8-methoxy-2-(4-methoxyphenyl)-3-methyl-3H-naphth[1,2-d]imidazole hydrochloride

The compound of the title, as the free base, is prepared by following essentially the same procedure described in example 4 but using 7-methoxy-2-methylamino-1-nitrosonaphthalene instead of 2-methylamino-1-nitrosonaphthalene and 4-methoxybenzaldehyde instead of 4-ethoxybenzal-

dehyde. By addition of HCl to a diethyl ether solution of the free base, the corresponding hydrochloride precipitates. Yield 40%. M.p. 265°C dec. (from methanol).

Example 28

7-methoxy-2-(4-methoxyphenyl)-3-methyl-3H-naphth[1,2-d]imidazole hydrochloride

The compound of the title is prepared by following essentially the same procedure described in the foregoing example but starting from 6-methoxy-2-methylamino-1-nitrosonaphthalene instead of 7-methoxy-2-methylamino-1-nitrosonaphthalene. Yield 35%. M.p. 261°C (from methanol).

Example 29

6-chloro-2-methoxy-2-(4-methoxyphenyl)-3-methyl-3H-naphth[1,2-d]imidazole

The compound of the title is prepared by following essentially the same procedure described in example 4 but starting from 5-chloro-6-methoxy-2-methylamino-1-nitrosonaphthalene instead of 2-methylamino-1-nitrosonaphthalene and employing 4-methoxybenzaldehyde instead of 4-ethoxybenzaldehyde. Yield 48%. M.p. 247—48°C (from ethanol).

Example 30

3-ethyl-2-(4-methoxyphenyl)-3H-naphth[1,2-d]imidazole

The compound of the title is prepared according to the procedure described in example 4 but starting from 2-ethylamino-1-nitrosonaphthalene instead of 2-methylamino-1-nitrosonaphthalene and adding 4-methoxybenzaldehyde instead of 4-ethoxybenzaldehyde. Yield 81%. M.p. 144—146°C (from ethyl acetate).

By operating according to the procedures of the foregoing examples the following compound may be prepared:

3-cyclopropyl-2-(4-methoxyphenyl)-3H-naphth[1,2-d]imidazole

*Preparation of the starting materials*

Example 31

N-isopropyl-1-nitroso-2-naphthaleneamine employed as the starting material in the process described in the second part of example 25 is prepared through reaction of 1-nitroso-2-naphthol with isopropylamine according to the method described in J. Am. Chem. Soc. *70*, 2415 (1948).

Example 32

The 6-methoxy-2-methylamino-1-nitrosonaphthalene employed in example 28 is prepared by adding 3.75 g of 6-methoxy-1-nitroso-2-naphthol to a solution of 9.5 cc of 35% $CH_3NH_2$ in 15 cc of water cooled to about 10°C, heating the reaction mixture to 40°C for a few minutes and finally recovering the solid which precipitates on cooling to room temperature.

Example 33

The 5-chloro-6-methoxy-2-methylamino-1-nitrosonaphthalene starting material of example 29 is prepared through reaction of methylamine with 5-chloro-6-methoxy-1-nitroso-2-naphthol following the procedure described in the foregoing example. In its turn this last compound is prepared by nitrosation of 5-chloro-6-methoxy-2-naphthol obtained from (5-chloro-6-methoxy-2-naphthalenyl)-ethanone which is a commercial product.

Example 34

2-Ethylamino-1-nitrosonaphthalene, starting material in example 30, is prepared by reacting 1-nitro-2-naphthol with ethylamine following the same procedure described in example 32.

Example 35

A tablet is prepared from

| | |
|---|---|
| 2-(4-methoxyphenyl-3-(isopropyl)--3H-naphth[1,2-d]imidazole | 500 mg |
| starch | 40 mg |
| talc | 10 mg |
| magnesium stearate | 10 mg |

Example 36

A tablet is prepared from

| | |
|---|---|
| 2-(4-methoxyphenyl)-3-methyl-3H-naphth[1,2-d]imidazole | 300 mg |
| lactose | 50 mg |
| microcrystalline cellulose | 50 mg |
| stearic acid | 10 mg |
| colloidal silica | 5 mg |

Example 37

A capsule is prepared from

| | |
|---|---|
| 4-(3-methyl-3H-naphth[1,2-d]imidazol-2-yl)-2,N,N-trimethylbenzenamine | 400 mg |
| talc | 40 mg |
| sodium carboxymethylcellulose | 40 mg |
| starch | 120 mg |

**Claims for the contracting States: BE CH DE FR GB IT LU NL SE**

1. A 3H-naphth[1,2-d]imidazole derivative having the following general formula I

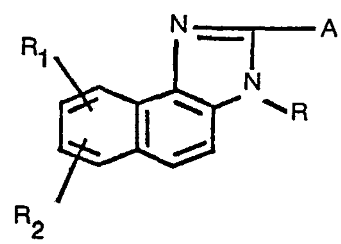

I

wherein R stands for $(C_1—C_6)$alkyl or cyclopropyl, $R_1$ and $R_2$, each independently, may represent hydrogen, $C_{(1-4)}$alkyl, halogen or $(C_{(1-4)}$alkoxy, and the symbol A represents a heteroaromatic ring selected from furyl, thienyl, pyrrolyl and pyridyl optionally substituted by $(C_1—C_4)$alkyl groups, or a phenyl radical optionally substituted with one to three groups independently selected from $(C_1—C_4)$alkyl, $(C_1—C_4)$alkoxy, amino, mono- and di-$(C_1—C_4)$alkylamino or with one methylenedioxy group, with the proviso that when simultaneously R stands for an ethyl radical, one of $R_1$ and $R_2$ is hydrogen and the other one is a methoxy group. A cannot be a 4-dimethylaminophenyl group, and salts therewith of pharmaceutically acceptable acids.

2. A compound as in claim 1 wherein R stands for $(C_{1-6})$alkyl, or cyclopropyl, $R_1$ and $R_2$ each independently represent hydrogen, $(C_1—C_4)$alkyl, halogen or $(C_{1-4})$alkoxy and the symbol A represents a phenyl radical optionally substituted with one to three groups independently selected from $(C_{1-4})$alkyl, $(C_{1-4})$alkoxy, amino, mono- and di$(C_{1-4})$alkylamino or with one methylenedioxy group; with the proviso that when simultaneously R stands for ethyl, one of $R_1$ and $R_2$ is hydrogen and the other is a methoxy group, A cannot represent a 4-dimethylaminophenyl group; and salts therewith of pharmaceutically acceptable acids.

3. A compound as in claim 1 wherein R stands for $(C_{1-6})$alkyl, $R_1$ and $R_2$ are hydrogen, and the symbol A represents a furyl, thienyl, pyrrolyl or pyridyl radical, optionally substituted by $(C_{1-4})$alkyl groups; and salts therewith of pharmaceutically acceptable acids.

4. A compound as in claim 1 wherein R stands for $(C_{1-6})$alkyl, $R_1$ and $R_2$ are both hydrogen and the symbol A represents a phenyl radical optionally substituted with one to three groups independently selected from $(C_{1-4})$alkyl, $(C_{1-4})$alkoxy, amino, mono- and di-$(C_{1-4})$alkylamino; and salts therewith of pharmaceutically acceptable acids.

5. A compound of claim 1 which is 2-(4-methoxyphenyl)-3-isopropyl)-3H-naphth[1,2-d]imidazole.

6. A compound of claim 1 which is 2-(4-methoxy phenyl)-3-methyl-3H-naphth[1,2-d]imidazole.

7. A compound of claim 1 which is 4-(3-methyl)-3H-naphth[1,2-d]imidazol-2-yl)-2,N,N-trimethylbenzenamine.

8. A compound of claim 1 for use as an antiinflammatory agent.

9. A composition suitable for antiinflammatory use which comprises from 50 to 1000 mg of a compound of claim 1, in admixture with a pharmaceutical carrier.

10. A compound of claim 1 for use as an antimicrobial agent.

11. A composition suitable for antimicrobial use which comprises from 0.1 percent by weight to 10 percent by weight of a compound of claim 1.

12. A process for preparing a compound of claim 1 characterized in that a N-substituted-1-nitroso-2-naphthaleneamine derivative of formula IV

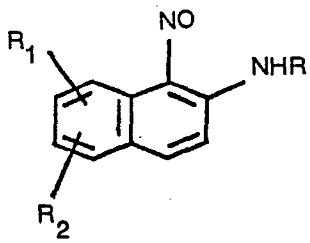

IV

wherein R, $R_1$ and $R_2$ are as defined in claim 1, is reduced to the corresponding $N^2$-substituted-naphthalen-1,2-diamine of formula II

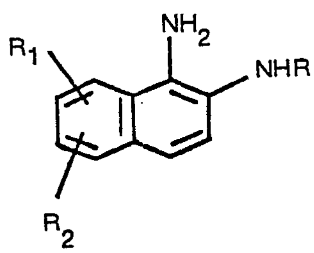

II

by means of hydrogen in the presence of a hydrogenation catalyst, said intermediate compound is condensed with a suitably selected aldehyde of formula ACHO wherein A is as defined in claim 1, to yield the corresponding imidazoline derivative of formula III

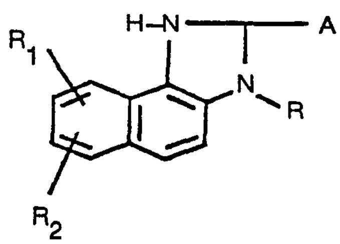

III

which in its turn is transformed into the final compound of formula I by means of a dehydrogenating agent; said process being further characterized in that

a) when a compound of formula I is desired wherein A stands for phenyl substituted with $(C_{1-4})$alkoxy, it may also be prepared from the corresponding compound of formula I wherein A stands for a hydroxyphenyl radical, through reaction with a suitable agent such as a $(C_{1-4})$alkyl halogenide, tosylate or mesylate,

b) when a compound of formula I is desired wherein A is phenyl substituted with amino, it may also be prepared from the corresponding compound of formula I wherein A is phenyl substituted with alkanoylamino or benzoylamino, by acid hydrolysis, and

c) when a compound of formula I is desired wherein A is phenyl substituted with mono-$(C_{1-4})$alkylamino, it may be also prepared from the corresponding compound of formula I wherein A is phenyl substituted with alkanoylamino or benzoylamino, by preparing the sodium derivative of the amidic

nitrogen atom, substituting it by means of an alkylating agent and then splitting off the protecting acyl group by alkaline hydrolysis.

**Claims for the contracting State: AT**

1. A process for preparing a 3H-naphth[1,2-d]imidazole derivative having the following general formula I

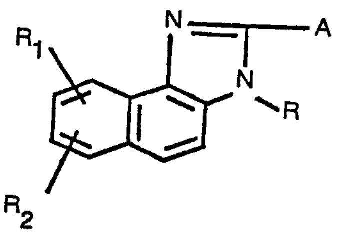

I

wherein R stands for ($C_1$—$C_6$)alkyl or cyclopropyl. $R_1$ and $R_2$, each independently, may represent hydrogen, $C_{(1-4)}$alkyl, halogen or ($C_1$—$C_4$)alkoxy, and the symbol A represents a heteroaromatic ring selected from fury, thienyl, pyrrolyl and pyridyl optionally substituted by ($C_1$—$C_4$)alkyl groups, or a phenyl radical optionally substituted with one to three groups independently selected from ($C_1$—$C_4$)alkyl, ($C_1$—$C_4$)alkoxy, amino, mono- and di-($C_1$—$C_4$)alkylamino or with one methylenedioxy group, with the proviso that when simultaneously R stands for an ethyl radical, one of $R_1$ and $R_2$ is hydrogen and the other one is a methoxy group. A cannot be a 4-dimethylaminophenyl group, and salts therewith of pharmaceutically acceptable acids characterized in that a $N^2$-substituted-naphthalen-1,2-diamine of formula II

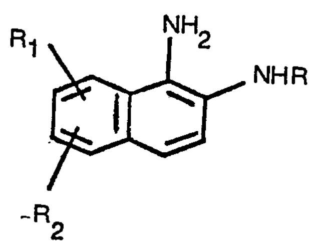

II

wherein R, $R_1$ and $R_2$ are as defined above, is condensed with a suitably selected aldehyde of formula ACHO wherein A is as defined above, to yield the corresponding imidazoline derivative of formula III

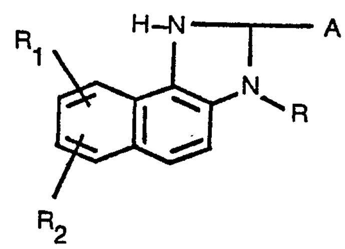

III

which in its turn is transformed into the final compound of formula I by means of a mild oxidizing agent or a dehydrogenating agent; said process being further characterized in that

a) when a compound of formula I is desired wherein A stands for phenyl substituted with ($C_{1-4}$)alkoxy, it may also be prepared from the corresponding compound of formula I wherein A stands for a hydroxyphenyl radical, through reaction with a suitable agent such as a ($C_{1-4}$)alkyl halogenide, tosylate or mesylate,

b) when a compound of formula I is desired wherein A is phenyl substituted with amino, it may also be prepared from the corresponding compound of formula I wherein A is phenyl substituted with alkanoylamino or benzoylamino, by acid hydrolysis, and

c) when a compound of formula I is desired wherein A is phenyl substituted with mono-($C_{1-4}$)alkylamino, it may be also prepared from the corresponding compound of formula I wherein A is phenyl

substituted with alkanoylamino or benzoylamino, by preparing the sodium derivative of the amidic nitrogen atom, substituting it by means of an alkylating agent and then splitting off the protecting acyl group by alkaline hydrolysis.

2. A process for preparing a compound as in claim 1 characterized in that a N-substituted-1-nitroso-2-naphthaleneamine derivative of formula IV

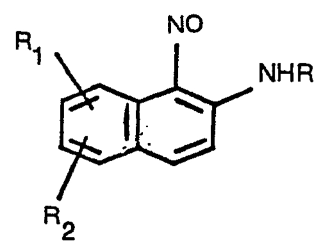

IV

wherein R, $R_1$ and $R_2$ are as defined in claim 1, is reduced to the corresponding $N^2$-substituted-naphthalen-1,2-diamine of formula II

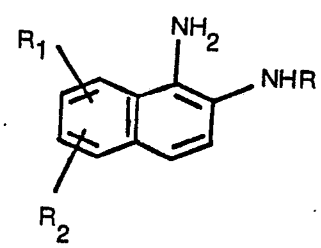

II

by means of hydrogen in the presence of a hydrogenation catalyst, said intermediate compound is condensed with a suitably selected aldehyde of formula ACHO wherein A is as defined in claim 1, to yield the corresponding imidazoline derivative of formula III

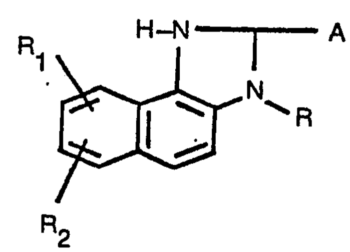

III

which in its turn is transformed into the final compound of formula I by means of a dehydrogenating agent; said process being further characterized in that

a) when a compound of formula I is desired wherein A stands for phenyl substituted with $(C_{1-4})$alkoxy, it may also be prepared from the corresponding compound of formula I wherein A stands for a hydroxyphenyl radical, through reaction with a suitable agent such as a $(C_{1-4})$alkyl halogenide, tosylate or mesylate,

b) when a compound of formula I is desired wherein A is phenyl substituted with amino, it may also be prepared from the corresponding compound of formula I wherein A is phenyl substituted with alkanoylamino or benzoylamino, by acid hydrolysis, and

c) when a compound of formula I is desired wherein A is phenyl substituted with mono-$(C_{1-4})$alkylamino, it may be also prepared from the corresponding compound of formula I wherein A is phenyl substituted with alkanoylamino or benzoylamino, by preparing the sodium derivative of the amidic nitrogen atom, substituting it by means of an alkylating agent and then splitting off the protecting acyl group by alkaline hydrolysis.

3. The process of claim 2 wherein the intermediates of formula II and III are not isolated from the reaction medium.

4. A process for preparing a compound as in claim 1 characterized in that a $N^2$-substituted naphthalen-1,2-diamine of formula II

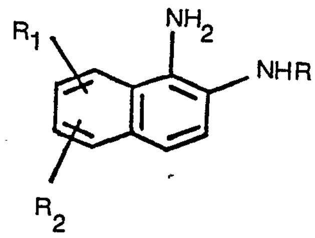

II

wherein R, $R_1$ and $R_2$ are as defined in claim 1, is condensed with an acid derivative of formula

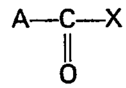

wherein A is as defined in claim 1 and X may represent
— a chlorine atom
— a group —$OR_3$ wherein $R_3$ may be the same radical

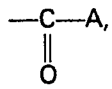

a trifluoroacetyl, ethoxycarbonyl or alkylsulphonyl moiety, or
— a group —$OR_4$ wherein $R_4$ stands for methyl or ethyl, to yield the corresponding mono-acylated compound of formula V

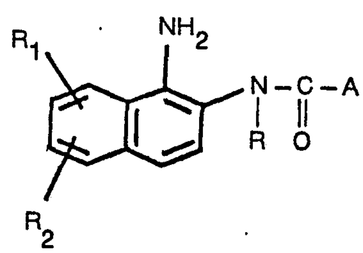

V

which in its turn is cyclized to the final compound of formula I; said process being further characterized in that
a) when a compound of formula I is desired wherein A stands for phenyl substituted with $(C_{1-4})$alkoxy, it may also be prepared from the corresponding compound of formula I wherein A stands for a hydroxyphenyl radical, through reaction with a suitable agent such as a $(C_{1-4})$alkyl halogenide, tosylate or mesylate,
b) when a compound of formula I is desired wherein A is phenyl substituted with amino, it may also be prepared from the corresponding compound of formula I wherein A is phenyl substituted with alkanoylamino or benzoylamino, by acid hydrolysis, and
c) when a compound of formula I is desired wherein A is phenyl substituted with mono-$(C_{1-4})$alkylamino, it may be also prepared from the corresponding compound of formula I wherein A is phenyl substituted with alkanoylamino or benzoylamino, by preparing the sodium derivative of the amidic nitrogen atom, substituting it by means of an alkylating agent and then splitting off the protecting acyl group by alkaline hydrolysis.

5. A process according to any of the preceding claims for preparing 2-(4-methoxyphenyl)-3-isopropyl-3H-naphth[1,2-d]imidazole.

6. A process according to any of claims 1, 2, 3 and 4 for preparing 2-(4-methoxyphenyl)-3-methyl-3H-naphth[1,2-d]imidazole.

7. A process according to any of claims 1, 2, 3, and 4 for preparing 4-(3-methyl-3H-naphth[1,2-d]imidazol-2-yl)-2,N,N-trimethylbenzenamine.

**Patentansprüche für die Vertragsstaaten: BE CH DE FR GB IT LU NL SE**

1. Ein 3H-Naphth[1,2-d]imidazolderivat der folgenden allgemeinen Formel I

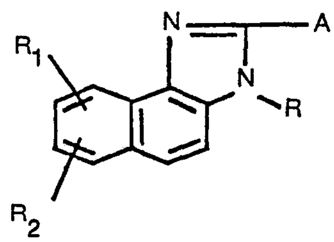

I

worin R für $(C_1—C_6)$Alkyl oder Cyclopropyl steht, $R_1$ und $R_2$ jeweils unabhängig voneinander Wasserstoff, $C_{(1-4)}$Alkyl, Halogen oder $C_{(1-4)}$Alkoxy bedeuten können und das Symbol A einen heteroaromatischen Ring, ausgewählt aus Furyl, Thienyl, Pyrrolyl und Pyridyl, der gegebenenfalls durch $(C_1—C_4)$Alkylgruppen substituiert ist, oder einen Phenylrest bedeutet, der gegebenenfalls durch ein bis drei Gruppen, welche unabhängig voneinander aus $(C_1—C_4)$Alkyl, $(C_1—C_4)$Alkoxy, Amino-, Mono- und Di$(C_1—C_4)$alkylamino ausgewählt sind, oder durch eine Methylendioxygruppe substituiert ist, mit der Maßgabe, daß, falls gleichzeitig R für einen Ethylrest steht, eines der Symbole $R_1$ und $R_2$ Wasserstoff ist und das andere eine Methoxygruppe ist, A nicht eine 4-Dimethylaminophenylgruppe sein kann, und dessen Salze mit pharmazeutisch annehmbaren Säuren.

2. Eine Verbindung nach Anspruch 1, worin R für $(C_{1-6})$Alkyl oder Cyclopropyl steht, $R_1$ und $R_2$ jeweils unabhängig voneinander Wasserstoff, $(C_1—C_4)$Alkyl, Halogen oder $(C_{1-4})$Alkoxy bedeuten und das Symbol A einen Phenylrest bedeutet, der gegebenenfalls durch eine bis drei Gruppen, die unabhängig voneinander aus $(C_{1-4})$Alkyl, $(C_{1-4})$Alkoxy, Amino, Mono- und Di$(C_{1-4})$alkylamino ausgewählt sind, oder durch eine Methylendioxygruppe substituiert ist; mit der Maßgabe, daß, falls gleichzeitig R für Ethyl steht, eines der Symbole $R_1$ und $R_2$ Wasserstoff ist und das andere eine Methoxygruppe ist, A nicht eine 4-Dimethylaminophenylgruppe bedeuten kann; und deren Salze mit pharmazeutisch annehmbaren Säuren.

3. Eine Verbindung nach Anspruch 1, worin R für $(C_{1-6})$Alkyl steht, $R_1$ und $R_2$ Wasserstoff sind und das Symbol A einen Furyl-, Thienyl-, Pyrrolyl- oder Pyridylrest bedeutet, der gegebenenfalls durch $(C_{1-4})$Alkylgruppen substituiert ist; und deren Salze mit pharmazeutisch annehmbaren Säuren.

4. Eine Verbindung nach Anspruch 1, worin R für $(C_{1-6})$Alkyl steht, sowohl $R_1$ als auch $R_2$ Wasserstoff sind und das Symbol A einen Phenylrest bedeutet, der gegebenenfalls durch eine bis drei Gruppen substituiert ist, welche unabhängig voneinander aus $(C_{1-4})$Alkyl, $(C_{1-4})$Alkoxy, Amino, Mono- und Di-$(C_{1-4})$alkylamino ausgewählt sind; und deren Salze mit pharmazeutisch annehmbaren Säuren.

5. Eine Verbindung nach Anspruch 1, die 2-(4-Methoxyphenyl)-3-isopropyl-3H-Naphth[1,2-d]imidazol ist.

6. Eine Verbindung nach Anspruch 1, welche 2-(4-Methoxyphenyl)-3-methyl-3H-naphth[1,2-d]imidazol ist.

7. Eine Verbindung nach Anspruch 1, welche 4-(3-Methyl-3H-Naphth[1,2-d]imidazol-2-yl)-2-N,N-trimethylbenzolamin ist.

8. Eine Verbindung nach Anspruch 1 zur Verwendung als ein entzündungshemmendes Mittel.

9. Eine zum entzündungshemmenden Gebrauch geeignete Zusammensetzung, enthaltend 50 bis 1000 mg einer Verbindung nach Anspruch 1, in Mischung mit einem pharmazeutischen Träger.

10. Eine Verbindung nach Anspruch 1 zur Verwendung als antimikrobielles Mittel.

11. Eine zur antimikrobiellen Anwendung geeignete Zusammensetzung, welche 0,1 Gew.-% bis 10 Gew.-% einer Verbindung nach Anspruch 1 enthält.

12. Ein Verfahren zur Herstellung einer Verbindung nach Anspruch 1, dadurch gekennzeichnet, daß ein N-substituiertes 1-Nitroso-2-naphthalinaminderivat der Formel IV

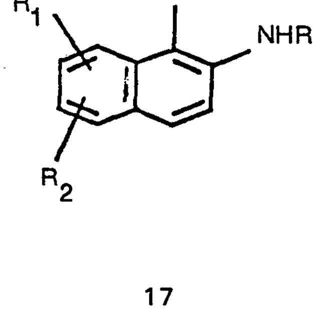

IV

worin R, $R_1$ und $R_2$ wie in Anspruch 1 definiert sind, mittels Wasserstoff in Gegenwart eines Hydrierungskatalysators zu dem entsprechenden $N^2$-substituierten Naphthalin-1,2-diamin der Formel II

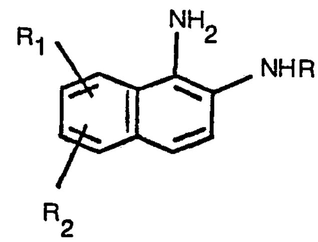

II

reduziert wird, diese Zwischenproduktverbindung mit einem geeignet ausgewählten Aldehyd der Formel ACHO, worin A wie in Anspruch 1 definiert ist, unter Bildung des entsprechenden Imidazolinderivats der Formel III

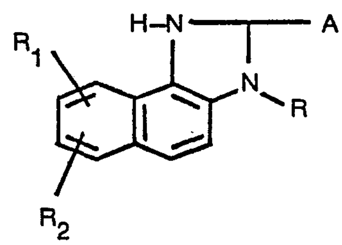

III

kondensiert wird, welches seinerseits mittels eines Dehydrogenierungsmittels in die Endverbindung der Formel I umgewandelt wird, welches Verfahren weiterhin dadurch gekennzeichnet ist, daß
a) falls eine Verbindung der Formel I erwünscht ist, worin A für durch $(C_{1-4})$Alkoxy substituiertes Phenyl steht, diese auch aus der entsprechenden Verbindung der Formel I, worin A für einen Hydroxyphenylrest steht, durch Umsetzung mit einem geeigneten Mittel, wie einem $(C_{1-4})$Alkylhalogenid, -tosylat oder -mesylat, hergestellt werden kann,
b) falls eine Verbindung der Formel I erwünscht ist, worin A durch Amino substituiertes Phenyl ist, diese auch aus der entsprechenden Verbindung der Formel I, worin A durch Alkanoylamino oder Benzoylamino substituiertes Phenyl ist, durch saure Hydrolyse hergestellt werden kann, und
c) falls eine Verbindung der Formel I erwünscht ist, worin A durch Mono-$(C_{1-4})$alkylamino substituiertes Phenyl ist, diese auch aus der entsprechenden Verbindung der Formel I, worin A durch Alkanoylamino oder Benzoylamino substituiertes Phenyl ist, unter Herstellung des Natriumderivats des Amidstickstoffatoms, Substitution desselben mittels eines Alkylierungsmittels und dann Abspaltung der Schutzacylgruppe mittels alkalischer Hydrolyse hergestellt werden kann.

**Patentansprüche für den Vertragsstaat: AT**

1. Ein Verfahren zur Herstellung eines 3H-Naphth[1,2-d]imidazolderivats der folgenden allgemeinen Formel I

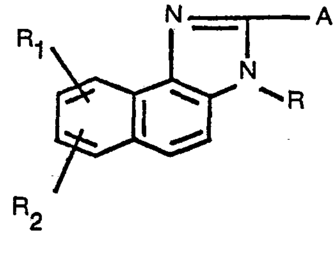

I

worin R für $(C_1—C_6)$Alkyl oder Cyclopropyl steht, $R_1$ und $R_2$ jeweils unabhängig voneinander Wasserstoff, $C_{(1-4)}$Alkyl, Halogen oder $C_{(1-4)}$Alkoxy bedeuten können und das Symbol A einen heteroaromatischen Ring, ausgewählt aus Furyl, Thienyl, Pyrrolyl und Pyridyl, der gegebenenfalls durch $(C_1—C_4)$Alkylgruppen substituiert ist, oder einen Phenylrest bedeutet, der gegebenenfalls durch ein bis drei Gruppen, welche unabhängig voneinander aus $(C_1—C_4)$Alkyl, $(C_1—C_4)$Alkoxy, Amino-, Mono- und Di$(C_1—C_4)$alkylamino ausgewählt sind, oder durch eine Methylendioxygruppe substituiert ist, mit der Maßgabe, daß, falls gleichzeitig R für einen Ethylrest steht, eines der Symbole $R_1$ und $R_2$ Wasserstoff ist

und das andere eine Methoxygruppe ist, A nicht eine 4-Dimethylaminophenylgruppe sein kann, und seiner Salze mit pharmazeutisch annehmbaren Säuren, dadurch gekennzeichnet, daß ein $N^2$-substituiertes Naphthalin-1,2-diamin der Formel II

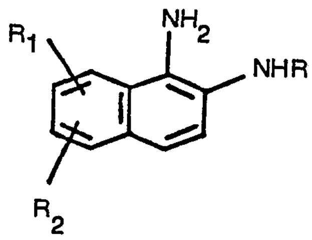

II

worin R, $R_1$ und $R_2$ wie oben definiert sind, mit einem geeignet ausgewählten Aldehyd der Formel ACHO, worin A wie oben definiert ist, unter Bildung des entsprechenden Imidazolinderivats der Formel III

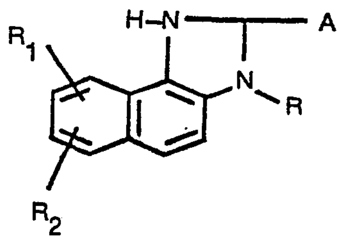

III

kondensiert wird, welches seinerseits mittels eines milden Oxydationsmittels oder eines Dehydrogenierungsmittels in die Endverbindung der Formel I umgewandelt wird, welches Verfahren weiterhin dadurch gekennzeichnet ist, daß

a) falls eine Verbindung der Formel I erwünscht ist, worin A für durch $(C_{1-4})$Alkoxy substituiertes Phenyl steht, diese auch aus der entsprechenden Verbindung der Formel I, worin A für einen Hydroxyphenylrest steht, durch Umsetzung mit einem geeigneten Mittel, wie einem $(C_{1-4})$Alkylhalogenid, -tosylat oder -mesylat, hergestellt werden kann,

b) falls eine Verbindung der Formel I erwünscht ist, worin A durch Amino substituiertes Phenyl ist, diese auch aus der entsprechenden Verbindung der Formel I, worin A durch Alkanoylamino oder Benzoylamino substituiertes Phenyl ist, durch saure Hydrolyse hergestellt werden kann, und

c) falls eine Verbindung der Formel I erwünscht ist, worin A durch Mono-$(C_{1-4})$alkylamino substituiertes Phenyl ist, diese auch aus der entsprechenden Verbindung der Formel I, worin A durch Alkanoylamino oder Benzoylamino substituiertes Phenyl ist, unter Herstellung des Natriumderivats des Amidstickstoffatoms, Substitution desselben mittels eines Alkylierungsmittels und dann Abspaltung der Schutzacylgruppe mittels alkalischer Hydrolyse hergestellt werden kann.

2. Ein Verfahren zur Herstellung einer in Anspruch 1 angegebenen Verbindung, dadurch gekennzeichnet, daß ein N-substituiertes 1-Nitroso-2-naphthalinaminderivat der Formel IV

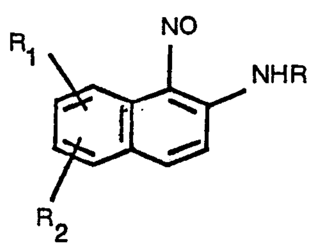

IV

worin R, $R_1$ und $R_2$ wie in Anspruch 1 definiert sind, mittels Wasserstoff in Gegenwart eines Hydrierungskatalysators zu dem entsprechenden $N^2$-substituierten Naphthalin-1,2-diamin der Formel II

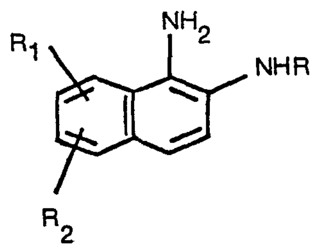

II

reduziert wird, diese Zwischenproduktverbindung mit einem geeignet ausgewählten Aldehyd der Formel ACHO, worin A wie in Anspruch 1 definiert ist, unter Bildung des entsprechenden Imidazolinderivats der Formel III

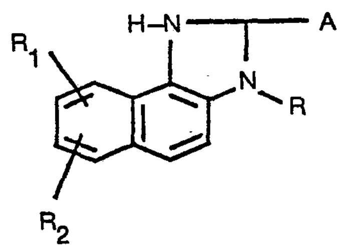

III

kondensiert wird, welches seinerseits mittels eines Dehydrogenierungsmittels in die Endverbindung der Formel I umgewandelt wird, welches Verfahren weiterhin dadurch gekennzeichnet ist, daß

a) falls eine Verbindung der Formel I erwünscht ist, worin A für durch $(C_{1-4})$Alkoxy substituiertes Phenyl steht, diese auch aus der entsprechenden Verbindung der Formel I, worin A für einen Hydroxyphenylrest steht, durch Umsetzung mit einem geeigneten Mittel, wie einem $(C_{1-4})$Alkylhalogenid, -tosylat oder -mesylat, hergestellt werden kann,

b) falls eine Verbindung der Formel I erwünscht ist, worin A durch Amino substituiertes Phenyl ist, diese auch aus der entsprechenden Verbindung der Formel I, worin A durch Alkanoylamino oder Benzoylamino substituiertes Phenyl ist, durch saure Hydrolyse hergestellt werden kann, und

c) falls eine Verbindung der Formel I erwünscht ist, worin A durch Mono-$(C_{1-4})$alkylamino substituiertes Phenyl ist, diese auch aus der entsprechenden Verbindung der Formel I, worin A durch Alkanoylamino oder Benzoylamino substituiertes Phenyl ist, unter Herstellung des Natriumderivats des Amidstickstoffatoms, Substitution desselben mittels eines Alkylierungsmittels und dann Abspaltung der Schutzacylgruppe mittels alkalischer Hydrolyse hergestellt werden kann.

3. Das Verfahren nach Anspruch 2, worin die Zwischenprodukte der Formeln II und III aus dem Reaktionsmedium nicht isoliert werden.

4. Ein Verfahren zur Herstellung einer Verbindung wie in Anspruch 1, dadurch gekennzeichnet, daß ein $N^2$-substituiertes Naphthalin-1,2-diamin der Formel II

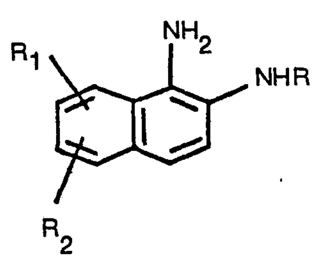

II

worin R, $R_1$ und $R_2$ wie in Anspruch 1 definiert sind, mit einem Säurederivat der Formel

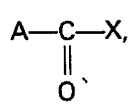

worin

A wie in Anspruch 1 definiert ist und X

— ein Chloratom

— eine Gruppe —$OR_3$, worin $R_3$ der gleiche Rest

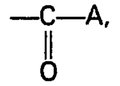

ein Trifluoracetyl-, Ethoxycarbonyl- oder Alkylsulfonylrest sein kann, oder
— eine Gruppe —$OR_4$, worin $R_4$ für Methyl oder Ethyl steht, bedeuten kann,
unter Bildung der entsprechenden mono-acylierten Verbindung der Formel V

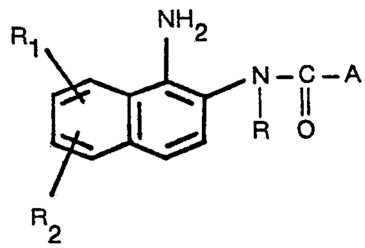

V

kondensiert wird, welche ihrerseits zur Endverbindung der Formel I cyclisiert wird; welches Verfahren weiterhin dadurch gekennzeichnet ist, daß
a) falls eine Verbindung der Formel I erwünscht ist, worin A für durch $(C_{1-4})$Alkoxy substituiertes Phenyl steht, diese auch aus der entsprechenden Verbindung der Formel I, worin A für einen Hydroxyphenylrest steht, durch Umsetzung mit einem geeigneten Mittel, wie einem $(C_{1-4})$Alkylhalogenid, -tosylat oder -mesylat, hergestellt werden kann,
b) falls eine Verbindung der Formel I erwünscht ist, worin A durch Amino substituiertes Phenyl ist, diese auch aus der entsprechenden Verbindung der Formel I, worin A durch Alkanoylamino oder benzoylamino substituiertes Phenyl ist, durch saure Hydrolyse hergestellt werden kann, und
c) falls eine Verbindung der Formel I erwünscht ist, worin A durch Mono-$(C_{1-4})$alkylamino substituiertes Phenyl ist, diese auch aus der entsprechenden Verbindung der Formel I, worin A durch Alkanoylamino oder Benzoylamino substituiertes Phenyl ist, unter Herstellung des Natriumderivats des Amidstickstoffatoms, Substitution desselben mittels eines Alkylierungsmittels und dann Abspaltung der Schutzacylgruppe mittels alkalischer Hydrolyse hergestellt werden kann.

5. Ein Verfahren nach einem der vorhergehenden Ansprüche zur Herstellung von 2-(4-Methoxyphenyl)-3-isopropyl-3H-naphth[1,2-d]imidazol.

6. Ein Verfahren nach einem der Ansprüche 1, 2, 3 und 4 zur Herstellung von 2-(4-Methoxyphenyl)-3-methyl-3H-naphth[1,2-d]imidazol.

7. Ein Verfahren nach einem der Ansprüche 1, 2, 3 und 4 zur Herstellung von 4-(3-Methyl-3H-naphth[1,2-d]imidazol-2-yl)-2,N,N-trimethylbenzolamin.

**Revendications pour les Etats contractants: BE CH DE FR GB IT LU NL SE**

1. Dérivé de 3H-naphto-[1,2-d]imidazole répondant à la formule générale I suivante:

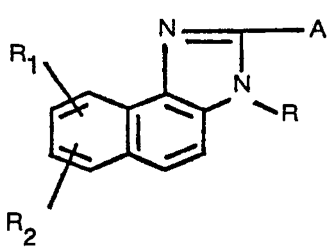

I

dans laquelle R représente un alkyle$(C_{1-6})$ ou un cyclopropyle, $R_1$ et $R_2$, chacun indépendamment, peuvent représenter un hydrogène, un alkyle en $C_{(1-4)}$, un halogène ou un alcoxy en $C_{(1-4)}$, le symbole A représente un cycle hétéro-aromatique choisi parmi furyle, thiényle, pyrrolyle et pyridyle éventuellement substitué par des groupes alkyles$(C_{1-4})$, ou un radical phényle éventuellement substitué par un à trois groupes choisis indépendamment parmi alkyle$(C_{1-4})$, alkoxy$(C_{1-4})$, amino, mono- et di-alkyl$(C_{1-4})$amino ou avec un groupe méthylènedioxy, sous réserve que, lorsque simultanément R représente un radical éthyle, un des symboles $R_1$ et $R_2$ est un hydrogène et l'autre est un groupe méthoxy, A ne peut pas être un groupe diméthylamino-4 phényle, et les sels correspondants formés avec des acides acceptables en pharmacie.

2. Composé comme dans la revendication 1, où R représente un alkyle($C_{1-6}$) ou un cyclopropyle, $R_1$ et $R_2$ représentent chacun indépendamment un hydrogène, un alkyle($C_{1-4}$), un halogène ou un alcoxy($C_{1-4}$) et le symbole A représente un radical phényle éventuellement substitué par un à trois groupes choisis indépendamment parmi un alkyle($C_{1-4}$), alcoxy($C_{1-4}$), amino, mono- et di-alkyl($C_{1-4}$)amino ou avec un groupe méthylènedioxy; sous réserve que, lorsque simultanément R représente un éthyle, un des symboles $R_1$ et $R_2$ représente un hydrogène et l'autre représente un groupe méthoxy, A ne peut pas représenter un groupe diméthylamino-4 phényle; et les sels correspondants formés avec des acides acceptables en pharmacie.

3. Composé selon la revendication 1, où R représente un alkyle($C_{1-6}$), $R_1$ et $R_2$ sont un hydrogène et le symbole A représente un radical furyle, thiényle, pyrrolyle ou pyridyle, éventuellement substitué par des groupes alkyles($C_{1-4}$); et les sels correspondants formés avec des acides acceptables en pharmacie.

4. Composé selon la revendication 1, où R représente un alkyle($C_{1-6}$), $R_1$ et $R_2$ sont tous deux un hydrogène et le symbole A représente un radical phényle éventuellement substitué par un à trois groupes choisis indépendamment parmi alkyle($C_{1-4}$), alcoxy($C_{1-4}$), mono- et di-alkyl($C_{1-4}$)amino; et les sels correspondants formés avec des acides acceptables en pharmacie.

5. Composé selon la revendication 1, qui est le (méthoxy-4 phényl)-2 isopropyl-3 3H-naphto[1,2-d]imidazole.

6. Composé selon la revendication 1, qui est le (méthoxy-4 phényl)-2 méthyl-3 3H-naphto[1,2-d]imidazole.

7. Composé selon la revendication 1, qui est la (méthyl-3 3H-naphto[1,2-d] imidazolyl-2)-4 N,N-diméthyl méthyl-2 benzènamine.

8. Composé selon la revendication 1, pour l'emploi comme agent anti-inflammatoire.

9. Composition convenant à l'emploi comme anti-inflammatoire qui comprend 50 à 1000 mg d'un composé selon la revendication 1, en mélange avec un véhicule pharmaceutique.

10. Composé selon la revendication 1, pour l'emploi comme agent antimicrobien.

11. Composition convenant à l'emploi antimicrobien qui comprend 0,1% en poids à 10% en poids d'un composé selon la revendication 1.

12. Procédé pour préparer un composé selon la revendication 1, caractérisé en ce que l'on réduit un dérivé de type nitroso-1 naphtalènamine-2 N-substituée de formule IV

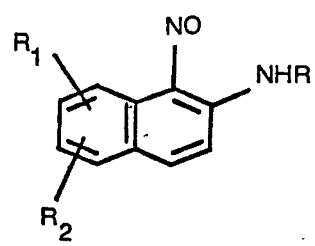

IV

dans laquelle R, $R_1$ et $R_2$ sont comme définis dans la revendication 1, en la naphtalènediamine-1,2 $N^2$-substituée de formule II

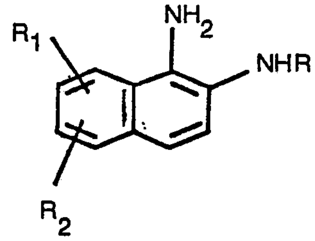

II

au moyen de l'hydrogène en présence d'un catalyseur d'hydrogénation, ledit composé intermédiaire est condensé avec un aldéhyde convenablement choisi de formule ACHO où A est comme défini dans la revendication 1, pour produire le dérivé d'imidazolyle correspondant de formule III

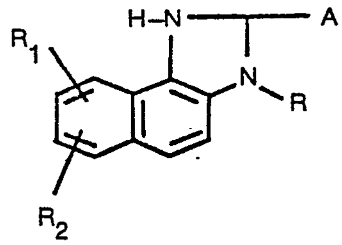

III

que l'on transforme à son tour en le composé final de formule I au moyen d'un agent de déshydrogénation; ledit procédé étant de plus caractérisé en ce que

a) lorsqu'on désire un composé de formule I où A représente un phényle substitué par un alcoxy($C_{1-4}$), on peut également le préparer à partir du composé correspondant de formule I où A représente un radical hydroxyphényle, par réaction avec un agent approprié tel qu'un halogénure, tosylate ou mésylate d'alkyle($C_{1-4}$),

b) lorsqu'on désire un composé de formule I où A est un phényle substitué par un amino, on peut également le préparer à partir du composé correspondant de formule I où A est un phényle substitué par un alcanoylamino ou un benzoylamino, par hydrolyse acide, et

c) lorsqu'on désire un composé de formule I où A est un phényle substitué par un mono-alkyl($C_{1-4}$)amino, on peut également le préparer à partir du composé correspondant de formule I où A est un phényle substitué par un alcanoylamino ou un benzoylamino, par préparation du dérivé de sodium de l'atome d'azote amidique, substitution de celui-ci par un agent d'alkylation puis clivage du groupe acyle protecteur par hydrolyse alcaline.

**Revendications pour l'Etat contractant: AT**

1. Procédé pour préparer un dérivé de 3H-naphto[1,2-d]imidazole répondant à la formule générale I suivante:

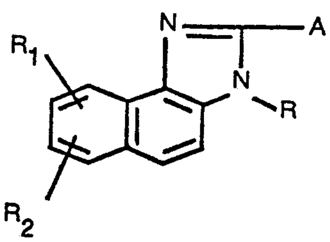

I

dans laquelle R représente un alkyle($C_{1-6}$) ou un cyclopropyle, $R_1$ et $R_2$, chacun indépendamment, peuvent représenter un hydrogène, un alkyle en $C_{(1-4)}$, un halogène ou un alcoxy en ($C_{1-4}$) et le symbole A représente un cycle hétéro-aromatique choisi parmi furyle, thiényle, pyrrolyle et pyridyle éventuellement substitué par des groupes alkyles($C_1$—$C_4$), ou un radical phényle éventuellement substitué par un à trois groupes choisis indépendamment parmi alkyle($C_1$—$C_4$), alkoxy($C_1$—$C_4$), amino, mono- et di-alkyl($C_1$—$C_4$)amino ou avec un groupe méthylènedioxy, sous réserve que, lorsque simultanément R représente un radical éthyle, un des symboles $R_1$ et $R_2$ est un hydrogène et l'autre est un groupe méthoxy, A ne peut pas être un groupe diméthylamino-4 phényle, et les sels correspondants formés avec des acides acceptables en pharmacie, caractérisé en ce qu'on condense une naphtalènediamine-1,2 $N^2$-substituée de formule II

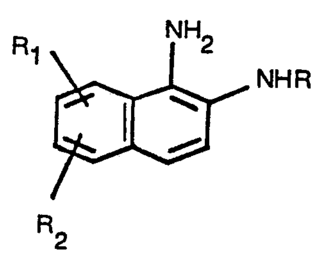

II

où R, $R_1$ et $R_2$ sont comme définis ci-dessus avec un aldéhyde convenablement choisi de formule ACHO où A est comme défini ci-dessus, pour fournir le dérivé d'imidazoline correspondant de formule III

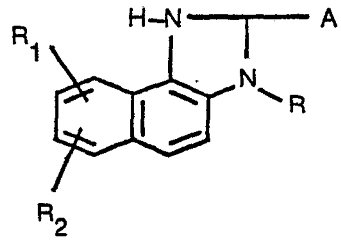

III

que l'on transforme à son tour en le composé final de formule I au moyen d'un agent oxydant doux ou d'un agent de déshydrogénation; ledit procédé étant de plus caractérisé en ce que:

a) lorsqu'on désire un composé de formule I où A représente un phényle substitué par un alcoxy($C_{1-4}$), on peut également le préparer à partir du composé correspondant de formule I où A représente un radical hydroxyphényle, par réaction avec un agent approprié tel qu'un halogénure, tosylate ou mésylate d'alkyle($C_{1-4}$),

b) lorsqu'on désire un composé de formule I où A est un phényle substitué par un amino, on peut également le préparer à partir du composé correspondant de formule I où A est un phényle substitué par un alcanoylamino ou un benzoylamino, par hydrolyse acide, et

c) lorsqu'on désire un composé de formule I où A est un phényle substitué par un mono-alkyl($C_{1-4}$)amino, on peut également le préparer à partir du composé correspondant de formule I où A est un phényle substitué par un alcanoylamino ou un benzoylamino, par préparation du dérivé de sodium de l'atome d'azote amidique, substitution de celui-ci par un agent d'alkylation puis clivage du groupe acyle protecteur par hydrolyse alcaline.

2. Procédé pour préparer un composé comme dans la revendication 1, caractérisé en ce que l'on réduit un dérivé de type nitroso-1 naphtalènamine-2 N-substitué de formule IV

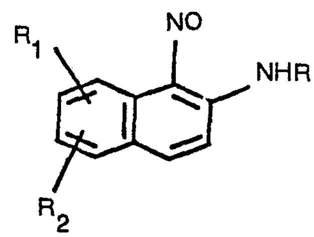

IV

où R, $R_1$ et $R_2$ sont comme définis dans la revendication 1, en la naphtalènediamine-1,2 $N^2$-substituée de formule II

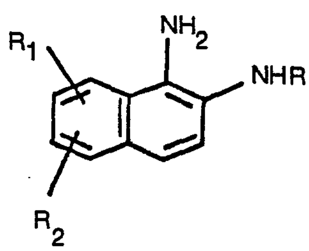

II

au moyen de l'hydrogène en présence d'un catalyseur d'hydrogénation, on condense ledit composé intermédiaire avec un aldéhyde convenablement choisi de formule ACHO où A est comme défini dans la revendication 1, pour produire le dérivé d'imidazoline correspondant de formule III

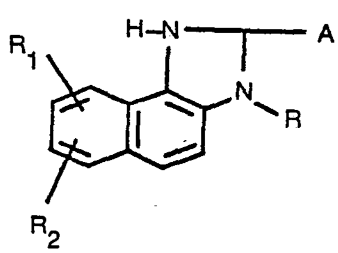

III

que l'on transforme à son tour en le composé final de formule I au moyen d'un agent de déshydrogénation; ledit procédé étant de plus caractérisé en ce que:

a) lorsqu'on désire un composé de formule I où A représente un phényle substitué par un alcoxy($C_{1-4}$), on peut également le préparer à partir du composé correspondant de formule I où A représente un radical hydroxyphényle, par réaction avec un agent approprié tel qu'un halogénure, tosylate ou mésylate d'alkyle($C_{1-4}$),

b) lorsqu'on désire un composé de formule I où A est un phényle substitué par un amino, on peut également le préparer à partir du composé correspondant de formule I où A est un phényle substitué par un alcanoylamino ou un benzoylamino, par hydrolyse acide, et

c) lorsqu'on désire un composé de formule I où A est un phényle substitué par un monoalkyl($C_{1-4}$)amino, on peut également le préparer à partir du composé correspondant de formule I où A est un phényle substitué par un alcanoylamino ou un benzoylamino, par préparation du dérivé de sodium de l'atome d'azote amidique, substitution de celui-ci par un agent d'alkylation puis clivage du groupe acyle protecteur par hydrolyse alcaline.

3. Procédé selon la revendication 2, dans lequel les intermédiaires de formule II et III ne sont pas isolés du milieu réactionnel.

4. Procédé pour préparer un composé comme dans la revendication 1, caractérisé en ce que l'on condense une naphtalène-diamine-1,2 $N^2$-substitué de formule II

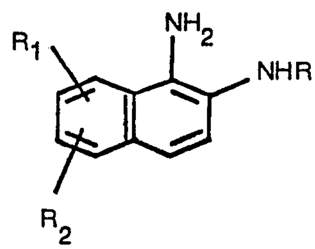

II

où R, $R_1$ et $R_2$ sont comme définis dans la revendication 1, avec un dérivé d'acide de formule

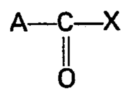

où A est comme défini dans la revendication 1, et X peut représenter

—un atome de chlore,

—un groupe —$OR_3$ où $R_3$ peut être le même radical

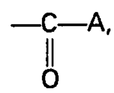

un fragment trifluoroacétyle, éthoxycarbonyle ou aklylsulfonyle, ou

—un groupe —$OR_4$ ou $R_4$ représente un méthyle ou un éthyle, pour obtenir le composé monoacylé correspondant de formule V

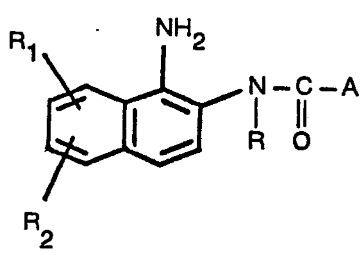

V

que l'on cyclise à son tour en le composé final de formule I; ledit procédé étant de plus caractérisé en ce que

a) lorsqu'on désire un composé de formule I où A représente un phényle substitué par un alcoxy($C_{1-4}$), on peut également le préparer à partir du composé correspondant de formule I où A représente un radical hydroxyphényle, par réaction avec un agent approprié tel qu'un halogénure, tosylate ou mésylate d'alkyle($C_{1-4}$),

b) lorsqu'on désire un composé de formule I où A est un phényle substitué par un amino, on peut également le préparer à partir du composé correspondant de formule I où A est un phényle substitué par un alcanoylamino ou un benzoylamino, par hydrolyse acide, et

c) lorsqu'on désire un composé de formule I où A est un phényle substitué par un mono-alkyl($C_{1-4}$)amino, on peut également le préparer à partir du composé correspondant de formule I où A est un phényle substitué par un alcanoylamino ou un benzoylamino, par préparation du dérivé de sodium de l'atome d'azote amidique, substitution de celui-ci par un agent d'alkylation puis clivage du groupe acyle protecteur par hydrolyse alcaline.

5. Procédé selon l'une quelconque des revendications précédentes, pour préparer le (méthoxy-4 phényl)-2 isopropyl-3 3H-naphto[1,2-d]imidazole.

6. Procédé selon l'une quelconque des revendications 1, 2, 3 et 4 pour préparer le (méthoxy-4 phényl)-2 méthyl-3 3H-naphto[1,2-d]imidazole.

7. Procédé selon l'une quelconque des revendications 1, 2, 3 et 4 pour préparer la (méthyl-3 3H-naphto[1,2-d]imidazolyl-2)-4 N,N-diméthyl méthyl-2 benzènamine.